# EUROPEAN PATENT APPLICATION

(11) **EP 1 426 069 A1**
(43) Date of publication of application: **09.06.2004**
(21) Application number: 02258307.4
(22) Date of filing: 02.12.2002
(51) Int. Cl.: A61M 5/34

(54) **Drug delivery system**

(71) Applicant: Miller, Diana Evelyn, Chippenham, Wiltshire SN14 6PS (GB); Capstick, John Brian, Barnes, London SW13 8DB (GB)
(72) Inventor: Miller, Diana Evelyn, Chippenham, Wiltshire SN14 6PS (GB); Capstick, John Brian, Barnes, London SW13 8DB (GB)
(74) Representative: Eyles, Christopher Thomas

(57) **Abstract**

An adaptor is described which is adapted for use in a drug delivery system for preventing inadvertent intrathecal administration of a drug. The adaptor comprises a hollow body (2;22) having a delivery end and a rear end opposite the delivery end; a standard male luer connection (15) at the delivery end for mating connection to a drug administration device (3) such as a cannula, needle, or catheter, that has a standard female luer fitting (19); a connection means (13) at the rear end for mating connection with a syringe (1) that contains the drug and has a delivery portion (10) with a delivery port (11) formed therein, the delivery portion (10) being sized and shaped so as to be incompatible with the standard female luer fitting (19) of the drug administration device (3); and a fluid passage (16) providing fluid communication between the standard male luer connection (15) and the connection means (13).

## Description

This invention relates to a drug delivery system, more particularly to a drug delivery system for intravenous administration of dangerous drugs, such as the vinca alkaloids, which can prove fatal if administered by intrathecal injection instead of by intravenous injection.

Protocols such as UKALL Trial XII, May 1995, recommend that cytotoxic therapy is given in cycles. Medication is often given on a semi-outpatient basis and often includes cytotoxic drugs, such as methotrexate or cytosine arabinoside, which are designed to be injected into the intrathecal space, i.e. the space containing the cerebrospinal fluid bathing the brain and spinal cord.

The vinca alkaloids, more particularly vinblastine, vincristine and vindesine, are another group of chemotherapeutic agents used in the treatment of leukemias, often in young people. They are designed to be given intravenously. If they are inadvertently injected into the intrathecal space, they cause ascending paralysis and death. There have been twenty two cases of inadvertent intrathecal injection of a vinca alkaloid reported around the world since 1968. Six have occurred in the United Kingdom between 1990 and 2001 and all were fatal.

Reports concerning some of these fatalities have been published as follows:-
1. *1997*/*8 - Reference Child's Nervous System ISSN: 0256-7040 (printed version) Abstract Volume 15 Issue 2*/*3 (1999) pp 87-89, Department of Neurosurgery, King Fahad National Guard Hospital, PO Box 22490, Riyadh 11426, Saudi Arabia - Ahmed A1 Ferayan, N A Russell, Mohammed A1 Wohaibi, Adnan Awada, Brian Scherman:*
2. *J Neuropathol Exp Neurol. 1968 Oct: 27(4):645-58 - Schochet S S, Lampert P W, Earle K M:*
3. *Med. Pediatr Oncol. 1978; 5(1): 85-8 - Shepherd D A, Steuber C P, Starling K A, Fernbach D J:*
4. *Neurology 1980 Aug; 30(8): 867-71 - Slyter H, Liwnicz B, Herrick M K, Mason R:*
5. *J Neurol 1982; 228(3): 209-13 - Manelis, Freudlich E, Ezekiel E, Doron J:*
6. *Cancer. 1983 June 1; 51(11): 2041-7; Williams M E, Walker A N, Bracikowski J P, Garner L, Wilson K D, Carpenter J T:*
7. *Cancer. 1983 Sep 1; 52(5): 788-801; Gaidys W G, Dickerman J D, Walters C L, Young P C:*
8. *N Engl J Med. 1989 Nov 2; 321(18): 1270-1; Dyke R W*
9. *BBC "Watchdog" 19.02.01-1990 case:*
10. *J Neurol 1991 July; 238(4): 230-4 - Bain P G, Lantos P L, Djurovic V; West J:*
11. *Clin Neuropharmacol 1991 Oct; 14(5): 457 - Bleck T P, Jacobsen J:*
12 . *Ann Trop Paediatr 1992; 12(3): 339-42 - al Fawaz I M*
13. *Med Pediatr Oncol 1995 Jan; 24(1): 61-2 - Zaragoza M R, Ritchey M L, Walter A:*
14. *Eur J Haematol 1997 Apr; 58(4): 286-8 - Geissler R G, Bergmann L, Hacker H, Knupp B, Walker B, Hoelzer D:*
15. *Br J Haematol 1997 Nov; 99(2): 364-7 - Michelagnoli M P, Bailey C C, Wilson I, Livingston J, Kinsey S E:*
16. *ISMP Medication Safety - Accidental administration of IV meds intrathecally - September 23, 1998 issue:*
17. *Clin Toxicol 1998; 36: 243-246 - Meggs W J et al.*
18. *J Pediatr Hematol Oncol 1998 Nov-Dec,. 20(6): 587-90 Fernandez C V, Esau R, Hamilton D, Fitzsimmons B, Pritchard S:*
19. *J Toxicol Clin Toxicol. 1998, 36(3): 243-6 - Meggs W J, Hoffman R S:*
20. *J Korean Med Sci. 1999 Dec; 14(6): 668-692 - Kwack E K,* *Kim D J, Park T I, Cho K R, Kwon I H, Sohn Y K:*
21. *Institute for Safe Medication Practices, ISMP Medication Safety (Pharmacists Society of the State of New York) 9 May 2000:*
22. *Medical Alerts! 2000-2001 Intrinsi Data Corporation www.intrinsiq.com*/*Med.Alerts:MedMain.htm*

There have also been reports of maladministration of other substances:-
(a) *Cancer. 1977 Dec; 40(6): 2817-22 - Breuer A C, Pitman S W; Dawson D M, Schoene W C:*

This report concerns paraparesis following intrathecal injection of cytosine arabinoside into a 65 year-old white male with acute myelogenous leukaemia.
(b) *Med Toxicol Adverse Drug Exp 1988 May-June; 3(3): 248-52 - Lafolie P, Liliemark J, Bjork 0, Aman* J, *Wranne L, Peterson C.*

This report deals with an accidental intrathecal overdose of cytarabine into a 4 year-old boy with acute lymphoblastic leukaemia.
(c) *Arch Phys Med Rehabil 1988 Dec; 69(12): 1054-6 - Werner R A:*

This decribes paraplegia and quadriplegia induced after intrathecal chemotherapy of methotrexate or cytosine arabinoside.
(d) *Med Pediatr Oncol. 1992; 20(3): 249-53 - Mortensen M E, Cecalupo A J, Lo W D, Egorin M J, Batley R:*

The effects of injection of dunorubican into a 3½ year old child with acute lymphoblastic leukaemia are described.

Most of the tragedies associated with vincristine have occurred when the substance has been inadvertently injected into the intrathecal space on the same occasion as one of the other drugs designed for intrathecal use.

Not surprisingly, the risk management effort has concentrated on finding ways to ensure that intrathecal drugs are kept separate from vinca alkaloids at every stage of the supply chain from pharmacy to patient. The focus has been primarily on protecting the supply chain for intrathecal drugs, rather than on restricting the movement of vincristine.

Much of the guidance and practice that has been developed emphasises increasingly elaborate arrangements to keep intrathecal drugs physically separate from drugs intended for administration by other routes. Professor K. Woods in a report dated April 2001, "The Prevention of Intrathecal Medication Errors", called for the publication of national guidelines on safety procedures to be followed in the administration of intrathecal chemotherapy by the National Health Service (hereafter "NHS"). In a report by Professor B. Toft, "External Inquiry into the adverse incident that occurred at Queen's Medical Centre, Nottingham, 4th January 2001", published in April 2001, calls are made for procedures for the dispensing, collection and transport of chemotherapy and for an explicit procedure for the administration of chemotherapy to be given to all members of the medical staff who administer intrathecal chemotherapy. Many of these recommendations have been incorporated in the document "National Guidance on the Safe Administration of Intrathecal Chemotherapy" which was sent out to NHS Trusts by the NHS Executive in November 2001.

While arrangements to insulate the intrathecal supply chain are certainly necessary, they can only ever be a partial solution to the problem of avoiding mix-ups with vinca alkaloids and other drugs intended for intravenous injection but which can have fatal consequences if inadvertently administered by the intrathecal route.

There is accordingly a need to develop a robust solution to the problem of preventing inadvertent administration of vinca alkaloids and other dangerous drugs by the wrong route.

The present invention accordingly seeks to provide a drug delivery system which obviates or substantially reduces the risk of maladministration of a vinca alkaloid by the incorrect method. It further seeks to provide a method of administering intravenously a dangerous drug, such as a vinca alkaloid, which, if inadvertently administered intrathecally instead of intravenously, can have fatal consequences.

According to one aspect of the present invention there is provided an adaptor adapted for use in a drug delivery system for preventing inadvertent intrathecal administration of a dangerous drug, the adaptor comprising;
a hollow body having a delivery end and a rear end opposite the delivery end;
a standard male luer connection at the delivery end for mating connection to a drug administration device that has a standard female luer fitting;
a connection means at the rear end for mating connection with a syringe that contains the drug and has a delivery portion with a delivery port formed therein, the delivery portion being sized and shaped so as to be incompatible with the standard female luer fitting of the drug administration device; and
a fluid passage providing fluid communication between the standard male luer connection and the connection means.

Examples of suitable drug administration devices are cannulae, needles and catheters.

In such an adaptor the connection means may comprise a female fitting having an internal conformation adapted for mating receipt of a male delivery portion of a syringe whose external conformation is oversize with respect to the standard luer fitting of the drug administration device.

Such an adaptor is intended for supply only to those members of the clinical staff who have been fully trained in the clinical use of vinca alkaloids, such as vincristine, or other dangerous drugs which can have potentially fatal consequences if administered via the incorrect route, for example, intrathecally. In parallel to use of an adaptor according to the invention within a hospital it will be ordained that vinca alkaloids, and any other drug which could have dangerous effects if inadvertently administered intrathecally, rather than intravenously, shall only be dispensed or otherwise provided to the clinical staff in specially designed syringes having a male non-luer fitting. Similarly it will be ordained that only needles, cannulae, or catheters having in each case a uniform standard female luer fitting will be used throughout the hospital. Since only a properly trained member of the clinical staff will be able to administer a vinca alkaloid or similar dangerous drug, because they will be the only persons authorised to possess such an adaptor, the risk of the vinca alkaloid or other dangerous drug being administered intrathecally will be minimised. On the other hand, drugs which do not exhibit such a potentially fatal effect if administered intrathecally instead of intravenously can continue to be dispensed within the hospital in normal syringes with standard male luer fittings which will fit matingly with a female luer fitting on a normal injection needle, catheter or cannula.

The hollow body of the adaptor will normally be configured so that it includes minimal dead space so that the fluid passage has as small an internal volume as possible.

Preferably the hollow body of the adaptor of the invention is provided adjacent its rear end with an external screw thread for screw threaded engagement with a corresponding internal screw thread on the syringe. It may also be provided with an external knurled surface to facilitate attachment of the adaptor to the syringe.

In one preferred form the passage provides unobstructed fluid communication between the connection means and the standard male luer fitting at the delivery end of the adaptor. Alternatively the passage may be provided with a diaphragm, membrane or filter. The purpose of such a diaphragm or membrane is to prevent back flow from the patient's vein in the case that the adaptor has been fitted to a cannula or catheter already inserted into a patient's vein, as well as to prevent ingress of infection or contamination of a member of the hospital staff in the extent of spillage.

Preferably the adaptor is provided with a distinctive colour, for example, red. It will usually have a body which is substantially rigid.

The invention further provides a drug delivery system for preventing inadvertent intrathecal administration of a drug solution, the delivery system comprising:
a drug delivery device having a delivery end and a female luer fitting at a rear end opposite the delivery end;
a syringe comprising a barrel with a rearward end and a forward end, and a plunger having a piston at a forward end thereof slidable in the barrel, the barrel having a male fitting at the forward end thereof which is incompatible with the female luer fitting on the drug delivery device; and
an adaptor comprising a hollow body having a back end and a front end, a female fitting at the back end adapted to receive the male fitting of the syringe, a male luer fitting at the front end adapted to be received in the female luer fitting of the drug delivery device, and a passage providing fluid communication between the female fitting and the male luer fitting.

In such a drug delivery system the female fitting of the adaptor preferably has an internal conformation adapted for mating receipt of the male fitting of the syringe and the male fitting of the syringe has an external conformation which is oversize with respect to the standard luer fitting of the drug administration device. Preferably the male fitting of the syringe is a non-luer fitting.

It is further preferred that the syringe or at least some prominently visible part thereof shall be coloured, for example, coloured red, so as' to provide a clear reminder to the nursing and other clinical staff that its contents comprise a dangerous drug, such as a vinca alkaloid.

In a preferred drug delivery system the syringe contains a unit dosage of a solution of a vinca alkaloid. The syringe with its unit dosage can be contained in a sealed package, possibly under sterile conditions. Alternatively the drug delivery system may further comprise a sealed container containing a solution of a vinca alkaloid, the container having in opened condition an opening permitting mating receipt of the male fitting of the syringe for filling the syringe with a unit dosage of the solution of the vinca alkaloid, and the opening having a conformation incompatible with a syringe having a standard male luer fitting.

Also provided in accordance with the invention is a method of effecting intravenous administration of a vinca alkaloid or other dangerous drug comprising the steps of:
(a) providing a drug delivery device having a delivery end and a female luer fitting at a rear end opposite the delivery end;
(b) providing a syringe containing a unit dosage of a solution of a vinca alkaloid or other dangerous drug, the syringe comprising a barrel with a rearward end and a forward end, and a plunger having a piston at a forward end thereof slidable in the barrel, the barrel having a male fitting at the forward end thereof which is incompatible with the female luer fitting on the drug delivery device;
(c) providing an adaptor comprising a hollow body having a back end and a front end, a female fitting at the back end adapted to receive the male fitting of the syringe, a male luer fitting at the front end adapted to be received in the female luer fitting of the drug delivery device, and a passage providing fluid communication between the female fitting and the male luer fitting;
(d) assembling the syringe to the adaptor by inserting, in either order, (i) the male fitting of the barrel in the female fitting of the adaptor and (ii) the male luer fitting of the adaptor in the female luer fitting of the drug delivery device; and
(e) introducing the unit dosage of the vinca alkaloid or other dangerous drug intravenously into a patient by means of the drug delivery device.

Preferably in such a method the drug delivery device comprises a cannula, needle, or catheter.

In a preferred method the syringe is supplied in prefilled form and is first removed from a sealed package, which may be sterile. Alternatively the syringe may be first filled from a sealed container containing a solution of a vinca alkaloid, the container having in opened condition an opening permitting mating receipt of the male fitting of the syringe for filling the syringe with a unit dosage of the solution of the vinca alkaloid, and the opening having a conformation incompatible with a syringe having a standard male luer fitting. The method may further include the step of drawing a quantity of sterile water into the syringe for dilution of the vinca alkaloid (or other dangerous drug) solution from a second container containing sterile water, the second container having in opened condition an opening permitting mating receipt of the male fitting of the syringe for drawing of sterile water into the syringe for dilution of the solution of the vinca alkaloid or other dangerous drug, the opening of the second container having a conformation incompatible with a syringe having a standard male luer fitting.

Also included within the scope of the invention is a method of preparing a drug delivery system including a drug delivery device in readiness for intravenous introduction of a vinca alkaloid into a patient which comprises steps (a) to (d) above.

The invention also relates to a container having in opened condition an opening permitting mating receipt of the male fitting of a syringe in accordance with the invention for filling the syringe from the container, and the opening having a conformation that is incompatible with a syringe having a standard male luer fitting.

In order that the invention may be clearly understood and readily carried into effect, a preferred embodiment thereof will now be described, by way of example only, with reference to the accompanying diagrammatic drawings, wherein:-
Figure 1 is an exploded perspective view of a first embodiment of a drug delivery system according to the invention, including a syringe, an adaptor and a cannula; and
Figure 2 is a similar perspective exploded view of a second form of drug delivery system in accordance with the invention with its adaptor shown in section.

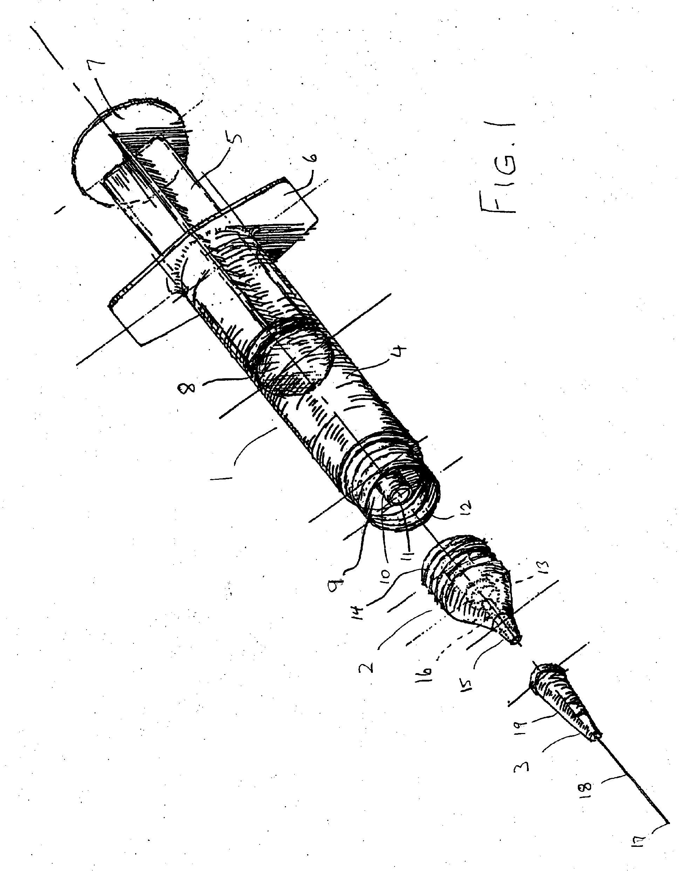

Referring to Figure 1 of the drawings, the drug delivery system comprises a syringe 1, an adaptor 2, and a cannula 3.

Syringe 1 is intended for use with an injectable solution containing a drug, such as vincristine, which is intended for intravenous injection and which has potentially fatal effects if inadvertently administered intrathecally. It may be supplied by the manufacturer in prefilled form, possibly in a sterilised sealed package. Alternatively it may be supplied empty and then filled with such an injectable solution in the hospital pharmacy by a suitably qualified pharmacist or at the patient's side by an appropriately qualified doctor or nurse from a suitable bottle or vial containing the drug solution. Dilution can, if necessary, be effected from a suitable second bottle or vial.

Syringe 1 includes a barrel 4 made of a transparent or translucent material, such as glass, nylon, or polyethylene, and a plunger 5. Barrel 4 is provided at its rear end with a flange 6 for engagement with the index and middle fingers of the hand of the user while plunger 5 has a flange 7 at its rearward end for engagement with the thumb of the user. At its forward delivery end plunger 5 has a piston 8 which is snugly received in the barrel 4. Adjacent its forward delivery end barrel 4 has a recessed transverse end flange 9 with an axial spigot 10 that provides an exit port 11 for the drug solution from barrel 4. If the syringe 1 is to be supplied in prefilled form, then the exit port 11 may be closed by a plastics cap or other suitable form of stopper that is removed prior to injection. On the other hand, if the syringe 1 is to be filled in the hospital pharmacy or at the patient's bedside, then exit port 11 has an open end through which syringe 1 may be filled from a bottle or capsule containing the drug solution, e.g. a vincristine solution. Such a bottle or capsule should have a filling opening, in its open condition, which can matingly receive the spigot 10 but which is incompatible with a standard luer male fitting of a conventional syringe.

The external surface of spigot 10 may be cylindrical or tapered. However, if it is tapered, it should have a non-standard diameter and/or taper so that it will not mate with a standard female luer connection. Preferably spigot 10 is oversized so that it cannot mate with a standard female luer connection. Barrel 4 is further provided adjacent its delivery end with an internal screw thread 12.

Adaptor 2 comprises a hollow body which is provided at its rearward end with an internal bore 13 which is adapted to mate with the axial spigot 10 and with an external screw thread 14 which is adapted for screw-threaded engagement with the internal screw thread of barrel 4. It is further provided at its forward delivery end with a standard male luer connector 15. A passageway 16, which may be provided with a diaphragm, membrane or filter, provides a passage for drug solution from exit port 11 to the forward end of male luer connector 15 when syringe 1 and adaptor 2 are assembled.

Cannula 3 is a standard cannula and has an open forward end 17 on a hollow shaft 18 and is provided at its rearward end with a standard female luer connection 19.

The syringe 1 and the cannula 3 can be aseptically packaged separately or in the same package. The adaptor 2 is aseptically packaged separately from the syringe 1 and the cannula 3.

In a hospital environment, a limited number only of adaptors 2 would be issued, and then each adaptor 2 would be issued only to a member of the nursing staff who has received proper training for administering intravenous and intrathecal chemotherapy. It is further envisaged that, in such an environment, it would be regulated that only needles, cannulae, and catheters having in each case a standard luer connection would be provided to the clinical staff for administration of injections of any sort. Moreover it would be regulated that a drug, such as vincristine, another vinca alkaloid or another drug which would have potentially fatal consequences if inadvertently administered intrathecally, is always packaged in a syringe 1 or is always dispensed in a syringe 1 by the hospital pharmacy. Under these circumstances, the only person who can administer that drug will be the authorised holder of an adaptor 2. In the absence of an adaptor 2, a non-authorised member of staff will not be able to fit a standard cannula, needle, or catheter to the filled syringe 1 and hence will not be able to inject the drug into a patient. In this way the risk of inadvertent intrathecal injection of vinca alkaloids and other drugs whose inadvertent administration by the intrathecal route could have potentially fatal results will be substantially obviated.

The risk can be further reduced by ensuring that only needles or cannulae or catheters or other fittings, such as nasogastric tubes, having in each case a single size of female luer fitting are used throughout the department or hospital and that, if more than one type of needle, cannula or catheter or other fitting is to be used, then each type should have the same size of female luer fitting.

The illustrated syringe 1 has a male fitting in the form of a cylindrical spigot 10. Alternatively spigot 10 could have another non-standard external profile. Thus, for example, spigot 10 could have a square, hexagonal, oval, star-shaped or other more complex cross-section for example, an oval cross section with two or more peripheral indentations, while adaptor 2 would in this case be provided with an internal bore 13 of corresponding mating cross-section. If spigot 10 is non-circular in section, then internal screw thread 12 will be omitted as will also external screw thread 14; in this case a captive locking ring with an internal screw thread can be provided on the forward end of barrel 4 or on adaptor 2 for engagement with a corresponding external screw thread on the adaptor 2 or on the forward end of barrel 4 so as to enable the adaptor 2 to be locked on syringe 1.

If desired, internal screw thread 12 of syringe 1 can be replaced by a smooth cylindrical surface, in which case adaptor 2 would be a push fit on the delivery end of syringe 1.

It may be expedient to colour adaptor 2, and possibly also syringe 1, red as a reminder to the user of the potential dangers of misadministration of the drug.

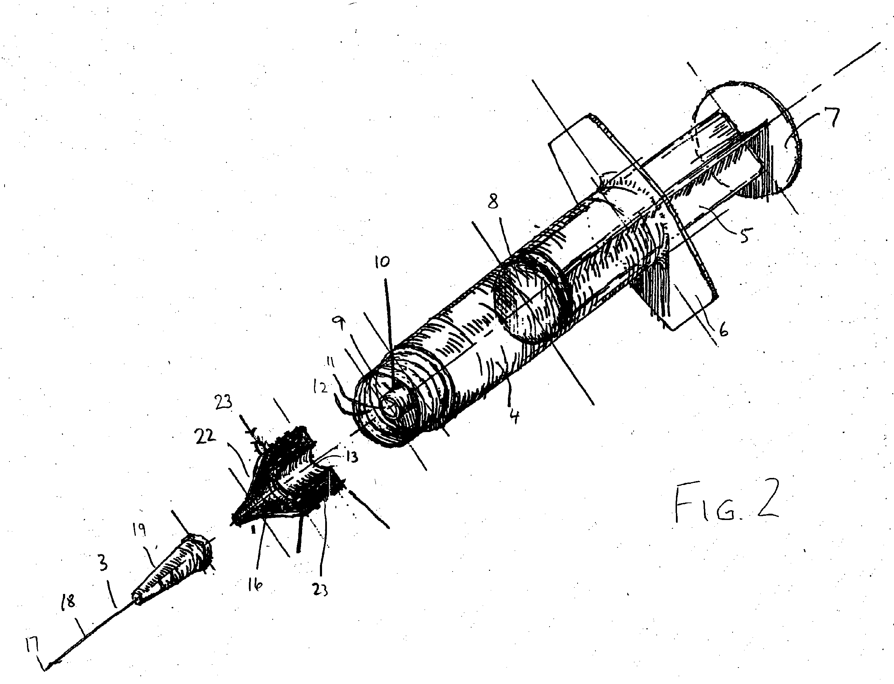

In the embodiment of Figure 2 the syringe 1 and cannula 3 are identical to those of Figure 1. However, adaptor 22 is provided with an external knurled surface 23 to facilitate manipulation thereof during assembly of the adaptor 22 to the syringe 1. This can be of particular relevance when the nurse or other member of the clinical staff is wearing gloves which may be wet.

Instead of having an external screw thread 24, adaptor 22 can have a plastic rim to engage the internal screw thread 12 of syringe 1.

Furthermore, if desired, adaptor 22 can be provided with a membrane or filter in order to prevent backflow from the patient's vein or infection or spillage and to take account of the move towards a needle-less infusion procedure. In patients who are being treated for any of the various forms of leukemia, there is often a very high risk of the introduction of infection. Similarly any spillage from backflow from the cannula during connection and disconnection of a device can result in contamination of a member of the hospital staff.

Instead of using a cannula 3, a needle or catheter can alternatively be used with either embodiment.

The capacity of the syringe 1 will be adapted to the unit dosage of the solution to be dispensed, e.g. 2 ml in the case of a prefilled syringe containing a vincristine solution. Alternatively, particularly if the syringe 1 is to be filled in the hospital dispensary or at the patient's bedside, barrel 4 can have a larger capacity than the expected unit dosage and can be graduated so that a larger volume than needed can be drawn into the syringe from an inverted bottle or vial having a mouth shaped to receive matingly the spigot 10 and then the excess volume expelled until the correct unit dosage volume remains in the syringe 1. If a paediatric patient is to be treated, then the volume of solution to be administered may be small. In this case it is expedient to provide also a second bottle or vial containing sterile water which also has a mouth shaped to receive matingly the spigot 10. Such a second bottle or vial may be of a different colour from the bottle or vial that contains the drug solution. For example, the sterile water can be contained in a bottle or vial which is clear and transparent whereas the vinca alkaloid or other drug is contained in a red coloured bottle or vial.

It is desirable that the syringe 1 shall be provided with appropriate calibration marks to enable the nurse or other member of the clinical staff to draw the correct volume of the solution of the vinca alkaloid or other dangerous drug into the syringe 1. Such calibration marks should be identified by clearly legible numbers written in the largest feasible font. This is to ensure that there shall be the minimal risk of inadvertent administration of an incorrect dosage, particularly a dosage that is too large in the case of a paediatric patient.

The female luer connection on the cannula, needle, or catheter can be of any standard size, e.g. 6.8 mm, 5.5 mm, or 4.1 mm.

The luer connections may be standard push-fit luer connections (or luer-slip connections) or luer lock connections.

## Claims

1. An adaptor adapted for use in a drug delivery system for preventing inadvertent intrathecal administration of a dangerous drug, the adaptor comprising;
a hollow body (2; 22) having a delivery end and a rear end opposite the delivery end;
a standard male luer connection (15) at the delivery end for mating connection to a drug administration device (3) that has a standard female luer fitting (19);
a connection means (13) at the rear end for mating connection with a syringe (1) that contains the drug and has a delivery portion (10) with a delivery port (11) formed therein, the delivery portion (10) being sized and shaped so as to be incompatible with the standard female luer fitting (19) of the drug administration device (3); and
a fluid passage (16) providing fluid communication between the standard male luer connection (15) and the connection means (13).

2. An adaptor according to claim 1, wherein the connection means (13) comprises a female fitting having an internal conformation adapted for mating receipt of a male delivery portion (10) of a syringe (1) whose external conformation is oversize with respect to the standard luer fitting (15) of the drug administration device (3).

3. An adaptor according to claim 1 or claim 2, wherein the hollow body (2; 22) is provided adjacent its rear end with an external screw thread (14) for screw threaded engagement with a corresponding internal screw thread (12) on the syringe (1).

4. An adaptor according to any one of claims 1 to 3, wherein the hollow body (22) is provided with an external knurled surface (23) to facilitate attachment of the adaptor to the syringe (1).

5. An adaptor according to any one of claims 1 to 4, wherein the passage (16) provides unobstructed fluid communication between the connection means (13) and the standard male luer fitting (15) at the delivery end of the adaptor.

6. An adaptor according to any one of claims 1 to 4, wherein the passage (16) is provided with a membrane or filter.

7. An adaptor according to any one of claims 1 to 6, wherein the body (2; 22) is coloured red.

8. An adaptor according to any one of claims 1 to 7, wherein the body (2; 22) is substantially rigid.

9. A drug delivery system for preventing inadvertent intrathecal administration of a drug solution, the delivery system comprising:
a drug delivery device (3) having a delivery end and a female luer fitting (19) at a rear end opposite the delivery end;
a syringe (1) comprising a barrel (4) with a rearward end and a forward end, and a plunger (5) having a piston (8) at a forward end thereof slidable in the barrel (4), the barrel (4) having a male fitting (10) at the forward end thereof which is incompatible with the female luer fitting (19) on the drug delivery device (3); and
an adaptor comprising a hollow body (2; 22) having a back end and a front end, a female fitting (13) at the back end adapted to receive the male fitting (10) of the syringe (1), a male luer fitting (15) at the front end adapted to be received in the female luer fitting (19) of the drug delivery device (3), and a passage (16) providing fluid communication between the female fitting (13) and the male luer fitting (15).

10. A drug delivery system according to claim 9, wherein the female fitting (13) of the adaptor has an internal conformation adapted for mating receipt of the male fitting (10) of the syringe (1) and wherein the male fitting (10) of the syringe (1) has an external conformation which is oversize with respect to the standard luer fitting (15) of the drug administration device (3).

11. A drug delivery system according to claim 9 or claim 10, wherein the hollow body (2; 22) is provided adjacent its rear end with an external screw thread (14) for screw threaded engagement with a corresponding internal screw thread (12) on the barrel (4) of the syringe (1).

12. A drug delivery system according to any one of claims 9 to 11, wherein the hollow body (22) is provided with an external knurled surface (23) to facilitate attachment of the adaptor to the barrel (4) of the syringe (1).

13. A drug delivery system according to any one of claims 9 to 12, wherein the passage (16) provides unobstructed fluid communication between the connection means (13) and the standard male luer fitting (15) at the delivery end of the adaptor.

14. A drug delivery system according to any one of claims 9 to 12, wherein the passage (16) is provided with a membrane or filter.

15. A drug delivery system according to any one of claims 9 to 14, wherein at least one out of the syringe (1) and the body (2; 22) is coloured red.

16. A drug delivery system according to any one of claims 9 to 15, wherein the body (2; 22) is substantially rigid.

17. A drug delivery system according to any one of claims 9 to 16, wherein the drug delivery device (3) comprises a cannula, needle, or catheter.

18. A drug delivery system according to any one of claims 9 to 17, wherein the syringe (1) contains a unit dosage of a solution of a vinca alkaloid.

19. A drug delivery system according to claim 18, wherein the syringe (1) is contained under sterile conditions in a sealed package.

20. A drug delivery system according to any one of claims 9 to 17, further comprising a sealed container containing a solution of a vinca alkaloid, the container having in opened condition an opening permitting mating receipt of the male fitting of the syringe for filling of the syringe (1) with a unit dosage of the solution of the vinca alkaloid, and the opening having a conformation incompatible with a syringe having a standard male luer fitting.

21. A drug delivery system according to claim 20, further including a second sealed container containing sterile water, the second sealed container having in opened condition an opening permitting mating receipt of the male fitting (10) of the syringe (1) for drawing of sterile water into the syringe (1) for dilution of the solution of the vinca alkaloid, the opening having a conformation incompatible with a syringe having a standard male luer fitting.

22. A method of effecting intravenous administration of a vinca alkaloid comprising the steps of:
(a) providing a drug delivery device (3) having a delivery end and a female luer fitting (19) at a rear end opposite the delivery end;
(b) providing a syringe (1) containing a unit dosage of a solution of a vinca alkaloid, the syringe (1) comprising a barrel (4) with a rearward end and a forward end, and a plunger (5) having a piston (8) at a forward end thereof slidable in the barrel (4), the barrel (4) having a male fitting (10) at the forward end thereof which is incompatible with the female luer fitting (19) on the drug delivery device (3);
(c) providing an adaptor comprising a hollow body (2; 22) having a back end and a front end, a female fitting (13) at the back end adapted to receive the male fitting (10) of the syringe (1), a male luer fitting (15) at the front end adapted to be received in the female luer fitting (19) of the drug delivery device (3), and a passage (16) providing fluid communication between the female fitting (13) and the male luer fitting (15);
(d) assembling the syringe (1) to the adaptor and the adaptor to the drug delivery device (3) by inserting, in either order, (i) the male fitting (10) of the barrel (4) in the female fitting (13) of the adaptor and (ii) the male luer fitting (15) of the adaptor in the female luer fitting (19) of the drug delivery device (3); and
(e) introducing the unit dosage of the vinca alkaloid intravenously into a patient by means of the drug delivery device (3).

23. A method according to claim 22, wherein the female fitting (13) of the adaptor has an internal conformation adapted for mating receipt of the male fitting (10) of the syringe (1) and wherein the male fitting (10) of the syringe (1) has an external conformation which is oversize with respect to the standard luer fitting (19) of the drug administration device (3).

24. A method according to claim 22 or claim 23, wherein the hollow body (2; 22) is provided adjacent its rear end with an external screw thread (14) for screw threaded engagement with a corresponding internal screw thread (12) on the barrel (4) of the syringe (1).

25. A method according to any one of claims 22 to 24, wherein the hollow body (22) is provided with an external knurled surface (23) to facilitate attachment of the adaptor to the barrel (4) of the syringe (1).

26. A method according to any one of claims 22 to 25, wherein the passage (16) provides unobstructed fluid communication between the connection means (13) and the standard male luer fitting (15) at the delivery end of the adaptor.

27. A method according to any one of claims 22 to 25, wherein the passage (16) is provided with a membrane or filter.

28. A method according to any one of claims 22 to 27, wherein at least one out of the syringe (1) and the body (2; 22) is coloured red.

29. A method according to any one of claims 22 to 28, wherein the body (2; 22) is substantially rigid.

30. A method according to any one of claims 22 to 29, wherein the drug delivery device (3) comprises a cannula, needle, or catheter.

31. A method according to any one of claims 22 to 30, wherein the syringe (1) is first removed from a sterile sealed package.

32. A method according to any one of claims 22 to 31, wherein the syringe (1) is first filled from a sealed container containing a solution of a vinca alkaloid, the container having in opened condition an opening permitting mating receipt of the male fitting of the syringe for filling the syringe (1) with a unit dosage of the solution of the vinca alkaloid, and the opening having a conformation incompatible with a syringe having a standard male luer fitting.

33. A method according to claim 31, further including drawing a quantity of sterile water into the syringe (1) for dilution of the solution of the vinca alkaloid from a second container containing sterile water, the second container having in opened condition an opening permitting mating receipt of the male fitting (10) of the syringe (1) for drawing of sterile water into the syringe (1) for dilution of the solution of the vinca alkaloid, the opening of the second container having a conformation incompatible with a syringe having a standard male luer fitting.
